# EUROPÄISCHE PATENTSCHRIFT

(11) **EP 3 671 207 B1**
(45) Veröffentlichungstag und Bekanntmachung des Hinweises auf die Patenterteilung: **07.07.2021**
(21) Anmeldenummer: 18214008.7
(22) Anmeldetag: 19.12.2018
(51) Int. Cl.: G01N 33/08, A01K 43/04, G01N 24/08

(54) **VERFAHREN ZUR IN-OVO GESCHLECHTSBESTIMMUNG VON VOGELEIERN**
METHOD FOR IN-OVO SEXING OF AVIAN EGGS
PROCÉDÉ DE DÉTERMINATION IN-OVO DU SEXE DES OISEAUX

(43) Veröffentlichungstag der Anmeldung: 24.06.2020
(73) Patentinhaber: Leibniz - Institut für Analytische Wissenschaften - ISAS - e.V., 44139 Dortmund (DE)
(72) Erfinder: HERGENRÖDER, Roland, 44229 Dortmund (DE); LAMBERT, Jörg, 48308 Senden (DE); TELFAH, Ahmad, 44137 Dortmund (DE)
(74) Vertreter: Meinke, Jochen

(56) Entgegenhaltungen:
- DE-A1-102013 205 426
- US-A1- 2006 279 281

## Beschreibung

Die Erfindung betrifft ein Verfahren zur in-ovo Geschlechtsbestimmung von befruchteten und bebrüteten Vogeleiern, bei welchem der Zeitverlauf des Bebrütens des jeweiligen Vogeleis bis zur Ausbildung wenigstens eines Blutgefäßes überwacht und anschließend das wenigstens eine Blutgefäß im Hinblick auf eine Geschlechtsbestimmung analysiert wird.

Jedes Jahr werden allein in Deutschland etwa 45 Millionen Hühnerküken kurz nach dem Schlüpfen getötet. Dabei handelt es sich um die männlichen Geschwister der modernen Legehennen. Von den Legehennen stammen unsere Konsumeier. Da für die Erzeugung von Fleisch andere Hühnerrassen besser geeignet sind, werden die Brüder der Legehennen in den meisten Fällen nicht aufgezogen, sondern getötet, was aus ethischen Gründen in Zukunft vermieden werden soll.

Es sind deshalb schon verschiedene Verfahren zur Geschlechtsbestimmung von befruchteten und bebrüteten Vogeleiern, insbesondere Hühnereiern, bekannt geworden, die jedoch bisher in der Praxis noch nicht eingesetzt werden.

Aus DE 10 2016 004 051 B3 ist ein Verfahren bekannt, das folgende Schritte aufweist:
- Überwachung des Zeitverlaufs des Bebrütens bis zur Ausbildung mindestens eines erkennbaren Blutgefäßes oder des Herzens mit fließendem Blut,
- Schaffung eines Lochs in der Kalkschale des Vogeleies mittels einer Locherzeugungseinheit,
- Suchen der sich im Ei ausbildenden Blutgefäße oder des Herzens mittels eines Visions-Systems und einer koaxialen oder lateralen Beleuchtung mit Licht im sichtbaren Wellenlängenbereich,
- Positionieren zumindest eines Blutgefäßes oder des Herzens in den Laserfokus mindestens einer Laserstrahlenquelle entweder durch Bewegen des Eies oder durch Bewegen eines den Laserfokus erzeugenden Objektivs,
- Bestrahlung des Blutgefäßes oder des Herzens mit mindestens einer eine Anregungswellenlänge emittierenden Laserstrahlenquelle,
- Registrieren der Rückstreustrahlung des bestrahlten Blutgefäßes oder des Herzens mittels mindestens eines Detektors, der mit mindestens einer mit einer nachgeschalteten Amplifikations- und Detektoreinheit verbundenen Auswerteeinheit in Verbindung steht, wobei während der Registrierung eine Bewegung des Blutgefäßes oder des Herzens aus dem Laserstrahl heraus durch Nachführung der Blutgefäße oder des Herzens oder des Objektivs erfolgen kann,
- Auswertung der Rückstreustrahlung einschließlich der Fluoreszenzstrahlung in der Auswerteeinheit aus der registrierten spektralen Intensität der Fluoreszenzstrahlung in einem zur Anregungswellenlänge rotverschobenen Spektralbereich, wobei die geschlechtsspezifischen Eigenschaften des männlichen Blutes und des weiblichen Blutes in der Intensität und im Spektralprofil der registrierten Fluoreszenzstrahlung enthalten sind und wobei zumindest eine der aus den gemessenen spektralen Intensitäten der Fluoreszenzstrahlung ermittelten Intensitätsgrößen oder deren zugeordnete Größen bezüglich des männlichen Blutes gegenüber zumindest einer der ermittelten Intensitätsgrößen oder deren zugeordnete Größen bezüglich des weiblichen Blutes in den Blutgefäßen oder im Herz einen auswertbaren unterschiedlichen Wert aufweisen,
- Bestimmung des Geschlechts des Vogeleies aus dem Unterschied zumindest eines der Werte der Fluoreszenzintensitätsgrößen oder deren zugeordnete Größen in der Auswerteeinheit und danach zumindest
- eine Anzeige des in der Auswerteeinheit bestimmten Geschlechts des Embryos im Vogelei.

Bei diesem Verfahren ist es somit erforderlich, ein Loch in der Kalkschale zu schaffen, was einerseits aufwendig ist und andererseits von Nachteil für die weitere Entwicklung des Embryos sein kann, wenn in das geöffnete Ei Fremdstoffe eindringen.

Aus US 6 029 080 A ist ein Verfahren zur in-ovo Geschlechtsbestimmung von befruchteten und bebrüteten Vogeleiern bekannt, bei welchem eine Vielzahl von Eiern bereitgestellt wird, danach die Geschlechtsorgane des Embryos mit Strahlungswellen für jedes der mehreren Eier durch Bewegung einer die Strahlung erzeugenden Vorrichtung oder der Eier bestrahlt werden, um ein nachweisbares Signal von den Geschlechtsorganen der Embryos zu erzeugen, und anschließend das Geschlecht des Embryos von jedem der Eier aus der Analyse des Signals bestimmt wird, wobei das Bestrahlen bevorzugt mittels Kernmagnetresonanz (NMR) erfolgt, und zwar mittels Magnetresonanzbildgebung (MRI), um ein visuelles Bild des Embryos zu erzeugen. Dieses Verfahren kann zwar an ungeöffneten Eiern durchgeführt werden, allerdings müssen die Embryos bereits so weit entwickelt sein, dass die Geschlechtsorgane ausgebildet und erkennbar sind. Zu diesem Zeitpunkt ist aber auch schon das Schmerzempfinden der Embryos ausgebildet, so dass ein anschließendes Töten männlicher Embryos aus ethischen Gründen unerwünscht ist. Außerdem ist die erforderliche Messtechnik (MRI) sehr aufwendig und kostenintensiv.

Ein Verfahren mit den Merkmalen des Oberbegriffes des Anspruchs 1 wird in DE 10 2013 205 426 A1 offenbart.

Es ist grundsätzlich auch bekannt, dass das Geschlecht eines sich entwickelnden Embryos invasiv anhand von Blutproben aus Eiern bestimmt werden kann. Dazu wird den Eiern am vierten Tag nach der Befruchtung eine Blutprobe entnommen und diese Blutprobe wird einer PCR (Polymerase-Ketten-Reaktion) unterworfen, um das Geschlecht eindeutig zu bestimmen. Weibliche Embryonen weisen W und Z Chromosomen auf und zeigen zwei Messbanden, während männliche Embryonen nur über Z Chromosomen verfügen und deshalb nur eine Messbande zeigen, so dass die Geschlechtsbestimmung eindeutig ist.

Aufgabe der Erfindung ist, ein nicht invasives Verfahren zur in-ovo Geschlechtsbestimmung von befruchteten und bebrüteten Vogeleiern zu schaffen, welches bereits wenige Tage nach Brutbeginn automatisiert mit möglichst geringem apparativen Aufwand durchgeführt werden kann.

Diese Aufgabe wird bei einem Verfahren der eingangs genannten Art erfindungsgemäß dadurch gelöst, dass das jeweilige Vogelei nach der Ausbildung wenigstens eines Blutgefäßes nicht invasiv von außen mittels der NMR-Spektroskopie vermessen wird, wobei das Verhältnis der Intensitäten der Signale der Nucleobasen aus der DNS des wenigstens einen Blutgefäßes zu den Signalen der aromatischen Aminosäuren Histidin, Phenylalanin und Tyrosin ermittelt wird, wobei dieses Intensitätsverhältnis für Blut aus männlichen und weiblichen Embryonen einen auswertbaren unterschiedlichen Wert aufweist, wobei anschließend das Geschlecht des jeweiligen Vogeleis von einer Auswerteeinheit aus dem ermittelten Intensitätsverhältnis bestimmt wird.

Es hat sich nun überraschend gezeigt, dass es möglich ist, das Geschlecht eines Embryos nach der Ausbildung eines Blutgefäßes, also bereits am vierten Bruttag, durch eine nicht invasive Vermessung mittels der NMR-Spektroskopie zu bestimmen. Dabei hat sich herausgestellt, dass zwischen männlichen und weiblichen Embryonen ein signifikanter Unterschied zwischen dem Verhältnis der Intensitäten der Signale der Nucleobasen aus der DNS des jeweiligen Blutgefäßes zu den Signalen der aromatischen Aminosäuren Histidin, Phenylalanin und Tyrosin besteht. Während das Intensitätsverhältnis bei männlichen Embryonen kleiner als 0,2 beträgt, beträgt es bei weiblichen Embryonen etwa 1:1. Diese Ergebnisse konnten mit herkömmlichen PCR-Messungen verifiziert werden. Damit ist es möglich, das Geschlecht eines Embryos nicht invasiv schon am vierten Bruttag zuverlässig zu bestimmen und anschließend männliche Embryonen nicht weiter zu bebrüten. Zu diesem Zeitpunkt hat der Embryo noch keine Nervenzellen entwickelt, d.h. das Aussortieren des bebrüteten Vogeleis ist ethisch zumindest weniger bedenklich.

Eine molekulare Interpretation der Geschlechtsbestimmung ist wegen der Komplexität des Untersuchungsgegenstandes schwierig. Das Genom der Blutzellen ist wegen des hohen Molekulargewichts einer direkten Untersuchung mit der NMR-Spektroskopie nicht zugänglich. Höchstwahrscheinlich stammen die gefundenen Signale von Nucleobasen aus kleinen DNS-Fragmenten (weniger als 10 Basenpaare), die mit der NMR Spektroskopie beobachtbar sind. Das legen Untersuchungen der Blutproben mit der zweidimensionalen Phosphor-NMR-Spektroskopie nahe. Die Existenz solcher "Zell-freien DNS" wird auch in der aktuellen Literatur diskutiert. Diese Marker bilden die Grundlage für eine zerstörungsfreie in-ovo-Bestimmung des Geschlechts von Vogeleiern basierend auf einem miniaturisierten NMR-Gerät.

In besonders bevorzugter Ausgestaltung ist deshalb vorgesehen, dass das jeweilige Vogelei in einem Niederfeld-NMR-Spektrometer angeordnet wird, wobei das Magnetfeld des Niederfeld-NMR-Spektrometers eine Magnetstärke zwischen 1 und 3 Tesla aufweist. Ein solches Niederfeld-NMR-Spektrometer hat sich als geeignet erwiesen, eine NMR-Messung an Vogeleiern zur Geschlechtsbestimmung durchzuführen. Ein NMR-Spektrometer bedarf nur eines vergleichsweise geringen apparativen und Kostenaufwandes, so dass das Verfahren auch für kleinere Brutbetriebe geeignet ist.

Ferner ist vorteilhaft vorgesehen, dass nur der obere Bereich des jeweiligen Vogeleis mit einem Hochfrequenzpuls beaufschlagt wird. Dazu wird das jeweilige Vogelei aufrechtstehend in einer Messordnung angeordnet, so dass sich die Blutgefäße des Embryos nach einer kurzen Standzeit im oberen Bereich des Eis befinden und dementsprechend lokalisiert mit dem NMR-Signal beaufschlagt werden können.

Dazu ist bevorzugt vorgesehen, dass auf die Oberseite des jeweiligen Vogeleis eine planare Hochfrequenzspule aufgelegt wird. Das Messobjekt, also das jeweilige Vogelei ist dann in einer Messanordnung aufgenommen, welche in einem homogenen äußeren Magnetfeld angeordnet ist. Die planare Hochfrequenzspule ist an der Oberseite des jeweiligen Vogeleis angeordnet und erzeugt das Radiofrequenzfeld für die NMR-Pulse. Gleichzeitig dient die planare Hochfrequenzspule, die ein stark inhomogenes Radiofrequenzfeld erzeugt, auch zur Lokalisierung des NMR-Signals. Eine Lokalisierung ist notwendig, damit hauptsächlich Signale aus dem oberen Bereich des Volumens aufgenommen werden, in dem sich die Blutgefäße des Embryos befinden.

In ganz besonders bevorzugter Ausgestaltung ist vorgesehen, dass mehrere Vogeleier gleichzeitig in mehreren nebeneinander angeordneten Niederfeld-NMR-Spektrometern angeordnet und sukzessive mit einem Hochfrequenzpuls beaufschlagt werden. Durch diese Ausgestaltung ist es möglich, in kürzerer Zeit parallel eine Geschlechtsbestimmung mehrerer Vogeleier durchzuführen.

Dabei ist dann vorteilhaft weiterhin vorgesehen, dass alle Niederfeld-NMR-Spektrometer mit einer gemeinsamen Auswerteinheit verbunden werden.

Die Erfindung ist nachstehend anhand der Zeichnung beispielshaft näher erläutert. Diese zeigt in
- Fig. 1: eine schematische, perspektivische Darstellung einer Messanordnung zur Durchführung des erfindungsgemäßen Verfahrens mit einem der Übersichtlichkeit halber oberhalb einer planaren Hochfrequenzspule angeordneten Vogelei, welches bei einer Messung tatsächlich unterhalb der Hochfrequenzspule angeordnet ist,
- Fig. 2: die Hochfrequenzspule der Messanordnung und
- Fig. 3: NMR-Spektren von sieben bebrüteten Vogeleiern.

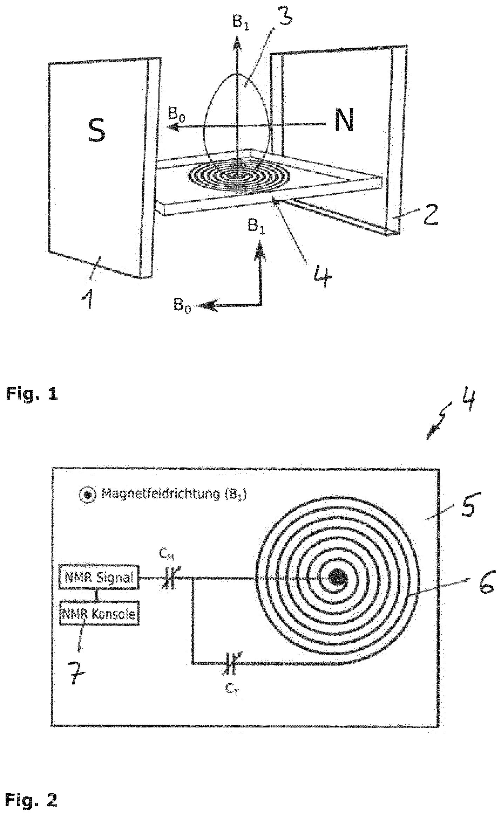

In Fig. 1 ist eine mögliche Messanordnung zur Durchführung des Verfahrens zur in-ovo Geschlechtsbestimmung von befruchteten und bebrüteten Vogeleiern dargestellt. Diese Messanordnung weist z.B. einem Halbach-NMR-Magneten auf, dessen beide Magnetplatten 1, 2 mit N (Nord) und S (Süd) gekennzeichnet sind. Dieser Magnet erzeugt in bekannter Weise ein homogenes äußeres Magnetfeld B₀. Innerhalb dieses homogenen Magnetfeldes B₀ zwischen den beiden Magnetplatten 1 und 2 ist eine nicht dargestellte, horizontal angeordnete Aufnahmeplatte aus nicht magnetischem Material angeordnet, auf welcher ein Vogelei 3 aufrechtstehend angeordnet wird. Der obere Bereich des Vogeleis 3 ist angrenzend zu einer horizontalen, planare Hochfrequenzspule 4 angeordnet. Diese planare Hochfrequenzspule ist der Übersichtlichkeit halber in Fig. 1 unterhalb des Vogeleis 3 dargestellt, tatsächlich befindet sie sich jedoch oberhalb und ist um 180° gedreht. Die Hochfrequenzspule 4 wird also tatsächlich von oben in Kontakt mit dem jeweiligen Vogelei 3 gebracht.

Die Hochfrequenzspule 4 liefert ein Radiofrequenzfeld B₁ zur Erzeugung der NMR-Pulse. Gleichzeitig dient die planare Hochfrequenzspule 4, die ein stark inhomogenes Hochfrequenz-RF-Feld B₁ erzeugt, auch zur Lokalisierung des NMR-Signales. Eine Lokalisierung ist notwendig, damit hauptsächlich Signale aus dem oberen Bereich des Vogeleis 3 aufgenommen werden, in dem sich die Blutgefäße des Embryos befinden, da sich diese bei aufrecht stehendem Vogelei 3 nach oben verlagern.

Fig. 2 zeigt den Aufbau der planaren Hochfrequenzspule 4. Auf einer Leiterplatte 5 ist eine Spule 6 über Kondensatoren (Match-Kondensator mit Kapazität C_{M} und Tune-Kondensator mit Kapazität C_{T}) mit einer NMR-Konsole 7 verbunden.

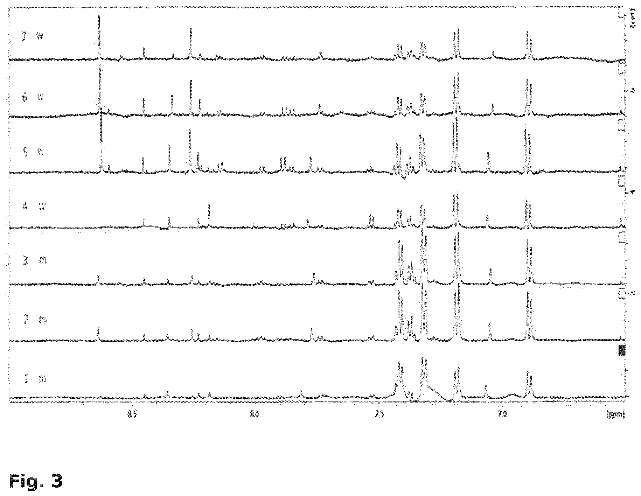

Fig. 3 zeigt die Spektren von sieben befruchteten und vier Tage lang bebrüteten Hühnereiern. Erkennbar sind im linken Bereich jeweils die Signale von Nucleobasen und im rechten Bereich jeweils die Signale der Aminosäuren Histidin, Phenylalanin und Tyrosin.

Die Signalhöhen der Nucleobasen im Vergleich zu den Signalhöhen der vorgenannten aromatischen Aminosäuren liegen für die Proben 4 bis 7 in derselben Größenordnung, während die Signalhöhen der Nucleobasen im Vergleich zu den Signalhöhen der vorgenannten aromatischen Aminosäuren für die Proben 1 bis 3 deutlich geringer sind. Daraus ergibt sich offensichtlich, dass sich aus dem Intensitätsverhältnis der Signale der Nucleobasen aus der DNS wenigstens eines Blutgefäßes eines befruchteten Embryos zu den Signalen der aromatischen Aminosäuren Histidin, Phenylalanin und Tyrosin für die Proben 4 bis 7 einerseits und für die Proben 1 bis 3 andererseits ein augenfällig unterschiedlicher Wert ergibt. Während bei den Proben 2 bis 3 das Intensitätsverhältnis sehr klein ist und etwa bei 0,2 liegt, liegt es bei den Proben 4 bis 7 etwa bei 1,0. Aufgrund dieses deutlichen Unterschiedes der Intensitätsverhältnisse konnte festgestellt werden, dass die Proben 1 bis 3 von männlichen Embryonen stammen und die Proben 4 bis 7 von weiblichen Embryonen. Diese Ergebnisse wurden mit herkömmlichem PCR (Polymerase-Ketten-Reaktion)-Bluttests derselben Proben verifiziert.

Das vorbeschriebene Verfahren lässt sich mit vergleichsweise geringem apparativen Aufwand und damit geringem Kostenaufwand durchführen, da für die NMR-Spektroskopie-Vermessung nur ein miniaturisiertes Niederfeld-Spektrometer notwendig ist, welches eine Magnetfeldstärke zwischen 1 und 3 Tesla aufweist. Bei Verwendung mehrerer parallel angeordneter Messanordnungen ist es möglich, mehrere Vogeleier quasi gleichzeitig in mehreren nebeneinander angeordneten Niederfeld-NMR-Spektrometern zu vermessen und sukzessive mit einem Hochfrequenzpuls zu beaufschlagen, was die Geschlechtsbestimmung von Vogeleiern für die Praxis mit einem akzeptablen Zeitaufwand durchführbar macht. So benötigt ein Messvorgang an einer Probe zwar etwa eine Minute, bei sukzessiver Hochfrequenzpulsbeaufschlagung mehrerer Messanordnungen lässt sich der Eierdurchsatz aber entsprechend beschleunigen.

### Bezugszeichenliste:

- 1: Magnetplatte
- 2: Magnetplatte
- 3: Vogelei
- 4: Hochfrequenzspule
- 5: Leiterplatte
- 6: Spule
- 7: NMR-Konsole

## Patentansprüche

1. Verfahren zur in-ovo Geschlechtsbestimmung von befruchteten und bebrüteten Vogeleiern, bei welchem der Zeitverlauf des Bebrütens des jeweiligen Vogeleis bis zur Ausbildung wenigstens eines Blutgefäßes überwacht und anschließend das wenigstens eine Blutgefäß im Hinblick auf eine Geschlechtsbestimmung analysiert wird, wobei das jeweilige Vogelei (3) nach der Ausbildung wenigstens eines Blutgefäßes nicht invasiv von außen mittels der NMR-Spektroskopie vermessen wird, **dadurch gekennzeichnet, dass** das Verhältnis der Intensitäten der Signale der Nucleobasen aus der DNS des wenigstens einen Blutgefäßes zu den Signalen der aromatischen Aminosäuren Histidin, Phenylalanin und Tyrosin ermittelt wird, wobei dieses Intensitätsverhältnis für Blut aus männlichen und weiblichen Embryonen einen auswertbaren unterschiedlichen Wert aufweist, wobei anschließend das Geschlecht des jeweiligen Vogeleis von einer Auswerteeinheit aus dem ermittelten Intensitätsverhältnis bestimmt wird.

2. Verfahren nach Anspruch 1,
**dadurch gekennzeichnet,**
**dass** das jeweilige Vogelei in einem Niederfeld-NMR-Spektrometer angeordnet wird, wobei das Magnetfeld des Niederfeld-NMR-Spektrometers eine Magnetfeldstärke zwischen 1 und 3 Tesla aufweist.

3. Verfahren nach Anspruch 2,
**dadurch gekennzeichnet,**
**dass** nur der obere Bereich des jeweiligen Vogeleis mit einem Hochfrequenzpuls beaufschlagt wird.

4. Verfahren nach Anspruch 3,
**dadurch gekennzeichnet,**
**dass** auf die Oberseite des jeweiligen Vogeleis eine planare Hochfrequenzspule aufgelegt wird.

5. Verfahren nach einem oder mehreren der Ansprüche 2 bis 4,
**dadurch gekennzeichnet,**
**dass** mehrere Vogeleier gleichzeitig in mehreren nebeneinander angeordneten Niederfeld-NMR-Spektrometern angeordnet und sukzessive mit einem Hochfrequenzpuls beaufschlagt werden.

6. Verfahren nach Anspruch 5,
**dadurch gekennzeichnet,**
**dass** alle Niederfeld-NMR-Spektrometer mit einer gemeinsamen Auswerteinheit verbunden werden.

## Claims

1. Method for in-ovo sexing of fertilised and incubated avian eggs, in which the progress over time of the incubation of the respective avian egg until the formation of at least one blood vessel is monitored and subsequently the at least one blood vessel is analysed with a view to sexing,
wherein the respective avian egg (3) is measured non-invasively from the outside by means of NMR spectroscopy after the formation of at least one blood vessel, **characterised in that** the ratio of the intensities of the signals of the nucleobases from the DNA of the at least one blood vessel to the signals of the aromatic amino acids histidine, phenylalanine and tyrosine is determined, wherein this intensity ratio has an evaluable different value for blood from male and female embryos, wherein subsequently the sex of the respective avian egg is determined by an evaluation unit from the determined intensity ratio.

2. Method according to claim 1,
**characterised in that**
the respective avian egg is arranged in a low-field NMR spectrometer, wherein the magnetic field of the low-field NMR spectrometer has a magnetic field strength between 1 and 3 Tesla.

3. Method according to claim 2,
**characterised in that**
only the upper region of the respective avian egg is subjected to a high-frequency pulse.

4. Method according to claim 3,
**characterised in that**
a planar high-frequency coil is placed on the upper side of the respective avian egg.

5. Method according to one or more of claims 2 to 4,
**characterised in that**
a plurality of avian eggs are simultaneously arranged in a plurality of low-field NMR spectrometers arranged side by side and successively subjected to a high-frequency pulse.

6. Method according to claim 5,
**characterised in that**
all low-field NMR spectrometers are connected to a common evaluation unit.

## Revendications

1. Procédé pour la détermination in ovo du sexe d'œufs d'oiseaux fécondés et incubés, dans lequel le déroulement du temps de l'incubation de l'œuf d'oiseau respectif jusqu'à la formation d'au moins un vaisseau sanguin est surveillé et ensuite l'au moins un vaisseau sanguin est analysé eu égard à une détermination du sexe,
dans lequel l'œuf d'oiseau respectif est mesuré de façon non invasive, de l'extérieur au moyen de la spectroscopie par RMN, après la formation d'au moins un vaisseau sanguin, **caractérisé en ce que** le rapport des intensités des signaux des bases nucléiques de l'ADN dudit au moins un vaisseau sanguin à celles des signaux des acides aminés aromatiques histidine, phénylalanine et tyrosine est déterminé, dans lequel ce rapport d'intensités présente une valeur différente évaluable pour du sang d'embryons mâles et celui d'embryons femelles, dans lequel le sexe de l'œuf d'oiseau respectif est ensuite déterminé par une unité d'évaluation à partir du rapport d'intensités déterminé.

2. Procédé selon la revendication 1,
**caractérisé en ce que**
l'œuf d'oiseau respectif est placé dans un spectromètre de RMN à champ proche, dans lequel le champ magnétique du spectromètre de RMN à champ proche présente une intensité de champ magnétique entre 1 et 3 teslas.

3. Procédé selon la revendication 2,
**caractérisé en ce que**
seulement la partie supérieure de l'œuf d'oiseau respectif est exposée à une impulsion à haute fréquence.

4. Procédé selon la revendication 3,
**caractérisé en ce qu'**
une bobine plane à haute fréquence est posée sur le côté supérieur de l'œuf d'oiseau respectif.

5. Procédé selon une ou plusieurs des revendications 2 à 4,
**caractérisé en ce que**
plusieurs œufs d'oiseaux sont agencés simultanément dans plusieurs spectromètres RMN à champ proche agencés les uns à côté des autres et sont exposés successivement à une impulsion à haute fréquence.

6. Procédé selon la revendication 5,
**caractérisé en ce que**
tous les spectromètres RMN à champ proche sont connectés à une unité d'évaluation commune.
